(19) Europäisches Patentamt
European Patent Office
Office européen des brevets

(11) Numéro de publication: **0 153 203**
**A1**

(12) **DEMANDE DE BREVET EUROPEEN**

(21) Numéro de dépôt: **85400059.3**

(22) Date de dépôt: **14.01.85**

(51) Int. Cl.⁴: **C 07 C 53/128**, C 07 C 53/126, C 07 C 57/03, A 61 K 31/19

(30) Priorité: **16.01.84 FR 8400586**

(43) Date de publication de la demande: **28.08.85 Bulletin 85/35**

(84) Etats contractants désignés: **AT BE CH DE FR GB IT LI LU NL SE**

(71) Demandeur: **P.F. MEDICAMENT, 125, Rue de la Faisanderie, F-75116 Paris (FR)**

(72) Inventeur: **Hatinguais, Philippe, Le Landou Chemin de Bel Air, F-81100 Castres (FR)**
Inventeur: **Negol, Paul, Route de Laprade, F-81290 Labruguière (FR)**
Inventeur: **Fahy, Jacques, 11, Avenue de Hauterive, F-81290 Labruguière (FR)**
Inventeur: **Carilla, Elisabeth "Les Magnolias", Appartement 04 48 Boulevard Léon Bourgeois, F-81100 Castres (FR)**

(74) Mandataire: **Ahner, Francis et al, CABINET REGIMBEAU 26, avenue Kléber, F-75116 Paris (FR)**

(54) **Acides gras ramifiés en C10 à C20 utiles en tant que médicaments, et leur procédé de préparation.**

(57) La présente invention concerne de nouveaux acides gras ramifiés en $C_{10}$ à $C_{20}$ utiles en tant que médicaments, et leur procédé de préparation.

Les acides gras ramifiés selon la présente invention répondent à la formule générale I

$$CH_3-(CH_2)_n-\underset{\displaystyle R}{\underset{|}{CH}}-(CH_2)_m-COOH \qquad (I)$$

dans laquelle:

R est un radical alcoyle ou alcényle linéaire ou ramifié contenant 1 à 3 atomes de carbone, et

les indices n et m sont des nombres entiers satisfaisant aux inégalités suivantes:

$$0 \leqslant n \leqslant 14$$
$$0 \leqslant m \leqslant 14$$
$$6 \leqslant n + m \leqslant 14$$

EP 0 153 203 A1

ACIDES GRAS RAMIFIES EN $C_{10}$ A $C_{20}$ UTILES EN TANT QUE MEDICAMENTS, ET LEUR PROCEDE DE PREPARATION.

La présente invention concerne la préparation et l'utilisation thérapeutique d'acides gras ramifiés en $C_{10}$ à $C_{20}$.

Alors que les acides gras linéaires, saturés ou insaturés, ont fait l'objet d'études approfondies et contribuent à caractériser les différentes huiles et graisses animales et végétales, en revanche les acides gras ramifiés sont généralement passés sous silence.

Cependant la présence de certains de ces acides gras à chaîne ramifiée a été signalée essentiellement dans les matières d'origine animale : acide isovalérique (HANSEN R.P. et coll. J.New Zeeland Inst. Chem. 1950, 14,142), acide 12-méthyltétradécanoïque, et acide 13-méthyltétradécanoïque (SHORLAND F.B. et coll. Biochem. J. 1955, 61,702), acides isobutyrique et α-méthylbutyrique (Mc INNES A.G. et coll. Biochem. J.1956,63,702).

Ces acides ramifiés ne représentent généralement qu'une très faible proportion, souvent inférieure à 1 %, des graisses et des huiles naturelles.

La demanderesse vient de découvrir de façon tout à fait surprenante que les acides gras ramifiés en $C_{10}$ à $C_{20}$ possédaient des propriétés thérapeutiques remarquables dans les domaines anti-inflammatoire, cardiovasculaire, antiprostatique et hypocholestérolémiant. De telles propriétés n'avaient

jamais pu être mises en évidence précédemment, du fait de la très forte dilution des acides gras ramifiés au sein d'ensembles complexes d'acides gras linéaires totalement inactifs ou bien du fait de leur présence à l'état naturel sous forme de dérivés inactifs, tels que les esters.

Les acides gras ramifiés en $C_{10}$ à $C_{20}$ entrant dans le cadre de la présente invention, répondent à la formule générale I

$$CH_3-(CH_2)_n-\underset{\displaystyle R}{\underset{|}{CH}}-(CH_2)_m-COOH \qquad (I)$$

dans laquelle :

R est un radical alcoyle ou alcényle linéaire ou ramifié contenant 1 à 3 atomes de carbone, et

les indices n et m sont des nombres entiers satisfaisant aux inégalités suivantes :

$$0 \leqslant n \leqslant 14$$

$$0 \leqslant m \leqslant 14$$

$$6 \leqslant n + m \leqslant 14$$

L'invention vise en particulier les acides gras ramifiés de formule générale I dans laquelle :

$R = -CH_3$ ou $-C_2H_5$

$n = 0$, et

$6 \leqslant m \leqslant 14$

La synthèse des acides gras ramifiés n'a jamais été réalisée dans un but de production industrielle, mais simplement de façon à obtenir des petits échantillons permettant des confirmations de structure ou quelques tests in vitro.

Le seul procédé de synthèse rencontré dans la littérature (SILVIUS J.R. et coll. Chemistry and Physics of lipids 1980, 26,67) permet d'accéder à l'acide antéisomyristique utilisé pour l'étude de diantéisoacylphosphotidyl choline

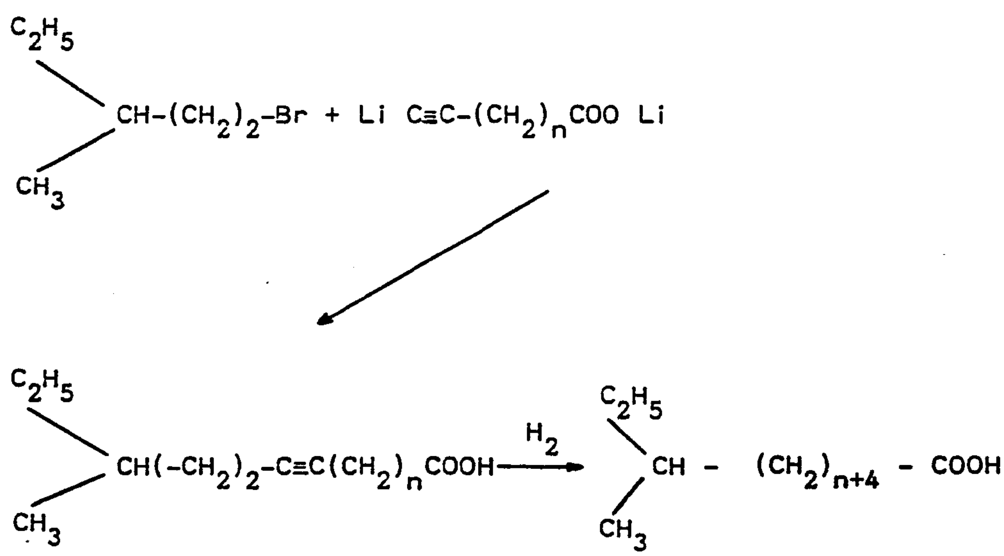

Cette synthèse nécessite l'emploi de matières premières coûteuses, difficiles à préparer, et conduit à des rendements variant de 5 à 80 % suivant les cas.

La demanderesse dans l'optique d'une préparation industrielle, a mis au point un procédé plus simple, mettant en oeuvre des matières premières courantes : halogéno-alcoyl magnésium, α, ω-dibromoalcane et malonate d'éthyl par exemple.

Selon la présente invention, les acides gras ramifiés de formule générale I sont préparés par réaction d'un halogéno-alcoyl magnésium de formule II

$$CH_3-(CH_2)_n-CH(MgX)-R \qquad (II)$$

où X représente un atome d'halogène,
avec un α, ω-dihalogénoalcane de formule III, en présence de tétrachlorocuprate de lithium,

$$X-(CH_2)_p-X \qquad (III)$$

où X représente un atome d'halogène et p = m ou m-1,
pour conduire à l'intermédiaire de synthèse de formule (IV)

$$CH_3-(CH_2)_n-\underset{\displaystyle R}{\underset{|}{CH}}-(CH_2)_p-X \qquad (IV)$$

où X et p ont la même signification que ci-dessus.

Dans ledit intermédiaire de synthèse de formule IV, la fonction halogène terminale est transformée en fonction acide carboxylique. Cette transformation peut par exemple être effectuée selon les deux variantes suivantes.

L'intermédiaire de formule IV où p = m-1 est mis à réagir avec du malonate d'éthyle et le diester ainsi obtenu est hydrolysé et décarboxylé pour conduire à l'acide gras ramifié correspondant de formule I. Cette première variante correspond au schéma réactionnel suivant :

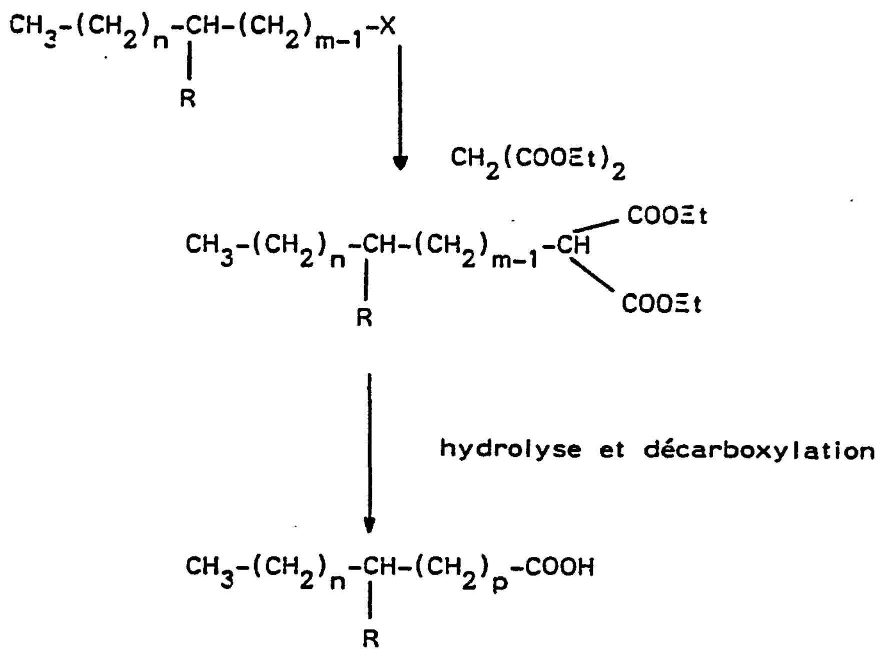

L'intermédiaire de formule IV où p = m est mis à réagir avec un cyanure de métal alcalin et le produit réactionnel est soumis à une hydrolyse conduisant à l'acide gras ramifié de formule I correspondant.

On mentionnera ci-après deux exemples de préparation d'acides gras ramifiés selon l'invention.

Exemple 1 :

Préparation de l'acide méthyl-11 tridécanoïque (acide antéiso-myristique)

On prépare une solution de bromo-2 butyl magnésium en dissolvant 9,85 g (0,41 mole) de magnésium dans 150 ml de THF sec, auxquels on ajoute 45,5 ml (0,41 mole) de bromo-2 butane.

Ce réactif est introduit lentement dans un mélange de 75 ml (0,41 mole) de dibromo-1,8 octane dans 150 ml de TMF à 0°C et de 0,2 ml d'une solution 0,1 M de tétrachlorocuprate de lithium. On laisse sous agitation pendant une heure.

Le bromo-1 méthyl-9 undécane est purifié par chromatographie sur colonne de silice éluée à l'hexane. On récupère ainsi 59 ml de produit.

Rendement : 56 %

On dissout 2,3 g (0,1 mole) de sodium dans 80 ml d'éthanol sec, auxquels on ajoute goutte à goutte 15,3 ml (0,1 mole) de malonate d'éthyle, puis 24 ml (0,1 mole) du dérivé bromé préparé précédemment.

On porte ensuite à reflux pendant 2 heures.

On distille alors sous pression réduite l'éthanol, et on lave l'ester formé par 3 fois 100 ml d'eau.

L'hydrolyse est réalisée en ajoutant 10,3 g (0,03 mole) d'ester dans 10 ml d'une solution 18 M de potasse que l'on porte à reflux pendant 2 h 30. Après refroidissement, on ajoute 10 ml d'acide sulfurique concentré au milieu, que l'on chauffe à reflux jusqu'à ce que la décarboxylation soit terminée, soit environ 3 heures.

La phase organique est extraite à l'hexane, traitée par un charbon actif,filtrée et concentrée. L'acide méthyl-11 tridécanoïque (5,4g) cristallise dans ce même solvant à -20°C.

Exemple 2 :

Préparation de l'acide méthyl-12 tridécanoïque (acide isomyristique)

En procédant de la même manière qu'à l'exemple 1, mais en utilisant au cours de la première étape le bromo-1 méthyl-2 propyl magnésium, on obtient le bromo-1 méthyl-10 undécane. La seconde étape de formation du diester par réaction avec le malonate d'éthyle puis la décarboxylation et l'hydrolyse conduisent finalement à l'acide isomyristique avec un rendement de 72 %.

L'étude pharmacologique conduite sur les acides gras ramifiés de formule générale I a permis de mettre en évidence leurs remarquables propriétés pharmacologiques permettant leur utilisation en tant que médicaments anti-prostatique, anti-inflammatoire, antiaggrégant plaquettaire, veinoconstricteur et hypocholestérolémiant. La présente demande vise donc également les compositions pharmaceutiques contenant, à titre de principe actif, au moins un des acides gras ramifiés de formule générale I, en association avec un support pharmaceutiquement acceptable.

Activité anti-inflammatoire

a) Une solution à 1 % d'acide isomyristique dans l'huile d'arachide (voie orale chez le rat) possède des propriétés anti-inflammatoires marquées mises en évidence dans le test de l'oedème généralisé au dextran, par action sur la phase vasculaire et diminution de l'aggrégation plaquettaire.

b) Le même type d'activité anti-inflammatoire a été mis en évidence, lors de l'administration par voie orale chez le rat, pour l'acide antéisomyristique ainsi que pour les acides gras ramifiés correspondants en $C_{12}$, $C_{16}$ et $C_{18}$.

## REVENDICATIONS

1/ A titre de médicaments nouveaux les acides gras ramifiés répondant à la formule générale I

$$CH_3-(CH_2)_n-\underset{\displaystyle R}{\underset{|}{CH}}-(CH_2)_m-COOH \qquad (I)$$

dans laquelle :

R est un radical alcoyle ou alcényle linéaire ou ramifié contenant 1 à 3 atomes de carbone, et

les indices n et m sont des nombres entiers satisfaisant aux inégalités suivantes :

$$0 \leqslant n \leqslant 14$$

$$0 \leqslant m \leqslant 14$$

$$6 \leqslant n + m \leqslant 14$$

2/ A titre de médicaments nouveaux les acides gras ramifiés répondant à la formule générale I selon la revendication 1, dans laquelle :

$$R = -CH_3$$

$$n = 0, \text{ et}$$

$$6 \leqslant m \leqslant 14$$

3/ A titre de médicaments nouveaux les acides gras ramifiés répondant à la formule générale I selon la revendication 1, dans laquelle :

$R = -C_2H_5$

$n = 0$, et

$6 \leq m \leq 14$

4/ A titre de médicaments nouveaux les acides gras ramifiés répondant à la formule générale I selon la revendication 1, dans laquelle :

$R = -CH_3$

$n = 0$, et

$m = 10$

5/ A titre de médicaments nouveaux les acides gras ramifiés répondant à la formule générale I selon la revendication 1, dans laquelle :

$R = -C_2H_5$

$n = 0$, et

$m = 9$

6/ Procédé de préparation de composés de formule générale I selon l'une des revendications 1 à 5, caractérisé en ce qu'en présence de tétrachlorocuprate de lithium, on fait réagir un halogéno-alcoyl magnésium de formule II

$$CH_3-(CH_2)_n-CH(MgX)-R \qquad (II)$$

avec un α, ω-dihalogénoalcane de formule III

$$X-(CH_2)_p-X \qquad (III)$$

où p = m ou m-1
pour conduire à l'intermédiaire de synthèse de formule IV

$$CH_3-(CH_2)_n-\underset{\displaystyle R}{\underset{|}{CH}}-(CH_2)_p-X \qquad (IV)$$

et en ce que, dans ledit intermédiaire de synthèse de formule IV, la fonction halogène terminale X est transformée en fonction acide carboxylique.

7/ Procédé selon la revendication 6, caractérisé en ce que l'on fait réagir l'intermédiaire de synthèse de formule IV où p = m-1 avec le malonate d'éthyle et en ce que le diester obtenu est hydrolysé et décarboxylé pour conduire à l'acide de formule générale I.

8/ Procédé selon la revendication 6, caractérisé en ce que l'on fait réagir l'intermédiaire de formule IV où p = m avec un cyanure de métal alcalin, et en ce que le produit réactionnel est soumis à une hydrolyse pour conduire à l'acide de formule générale I.

9/ Composition pharmaceutique, caractérisée en ce qu'elle contient à titre de principe actif au moins un acide gras ramifié de formule générale I selon la revendication 1, ainsi qu'un support pharmaceutiquement acceptable.

0153203

Office européen des brevets

# RAPPORT DE RECHERCHE EUROPEENNE

Numéro de la demande

EP 85 40 0059

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 005 422 (LABAZ)<br>* Revendications 1,13-15 * | 1,9 | C 07 C 53/128<br>C 07 C 53/126<br>C 07 C 57/03<br>A 61 K 31/19 |
| Y | FR-A-2 276 036 (LABAZ)<br>* Revendications 1,16,20,23,25-28 * | 1,9 | |
| Y | CHEMICAL ABSTRACTS, vol. 96, no. 14, 5 avril 1982, page 387, réf. no. 110139s, Columbus, Oio, US; & JP - A - 81 154 414 (TSURUHARA SEIYAKU K.K.) 30.11.1981<br>* Résumé * | 1 | |
| A | US-A-3 035 987 (WEITZEL)<br>* Revendication 2; colonne 2, lignes 40-44 * | 1 | |
| | | | **DOMAINES TECHNIQUES RECHERCHES (Int. Cl.4)** |
| | | | C 07 C 53/00<br>C 07 C 57/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 15-04-1985 | KLAG M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503. 03.82